# EUROPEAN PATENT SPECIFICATION

(11) **EP 0 417 325 B1**
(45) Date of publication and mention of the grant of the patent: **11.07.2001**
(21) Application number: 90905643.4
(22) Date of filing: 03.04.1990
(51) Int. Cl.: C07C 255/47, C07C 253/10

(54) **PROCESS FOR PRODUCING NORCAMPHANEDICARBONITRILES**
VERFAHREN ZUR HERSTELLUNG VON NORCAMPHANDIKARBONITRILEN
PROCEDE POUR PRODUIRE DES NORCAMPHANEDICARBONITRILES

(30) Priority: 04.04.1989 JP 8397089
(43) Date of publication of application: 20.03.1991
(73) Proprietor: MITSUI CHEMICALS, INC., Tokyo (JP)
(72) Inventor: INOMATA, Masamitu, Chiba 297 (JP); SHIOTANI, Naokazu, Chiba 297 (JP); KOSHIZUKA, Kazuo, Chiba 297 (JP); KARASAWA, Minato, Chiba 297 (JP)
(74) Representative: Luderschmidt, Schüler & Partner GbR
(86) International application number: JP9000450
(87) International publication number: WO9011998

(56) References cited:
- JP-A- 57 156 454
- JP-A- 57 193 438
- US-A- 3 496 217
- US-A- 3 655 723
- US-A- 3 766 237
- AMER. CHEM. SOC. DIV. PETROL. CHEM. PREPRINTS vol. 14, no. 2 , 1969 pages B29 - B34 E. S. BROWN ET AL 'THE PALLADIUMCATALYZED ADDITION OF HCN TO OLEFINS'

## Description

### Technical Field

The present invention relates to a process for producing a norcamphane dicarbonitrile (hereinafter referred to as "NDC") compound, and more particularly, to a process for producing a norcamphane dicarbonitrile compound mainly composed of 2,5-norcamphane dicarbonitrile (hereinafter referred to as "2,5-NDC") and 2,6-norcamphane dicarbonitrile (hereinafter referred to as "2,6-NDC") comprising hydrocyanation of bicyclo [2,2,1]-5-heptene-2-carbonitrile (hereinafter referred to as "BHC").

### Background Art

In the past only the following processes for producing NDC's by hydrocyanation of BHC were known :
(i) A process where a catalyst system comprising a cobalt carbonyl catalyst and triphenyl phosphine is used (see U.S. Patent Nos. 2,666,780 and 2,666,748),
(ii) A process where a catalyst system composed of tetrakis (triaryl phosphite) palladium and triphenyl phosphite is used (Am. Chem. Soc. Div. Pet. Chem. Preprints, 14, B 29 (1969), etc.
(iii) U.S. Patent No. 3,496,217 discloses a process for producing dinitriles such as adiponitrile and the like which comprises hydrocyanation of olefins such as 3-pentenenitrile, 4-pentenenitrile and the like in the presence of a zero-valent nickel complex and a promoter such as zinc chloride and the like.
(iv) U.S. Patent Nos. 3,655,723 and 3,766,237 disclose examples where a zero-valent nickel complex and zinc chloride are used upon hydrocyanation of methyl bicyclo (2,2,1)-5-heptene-2-carboxylate as a bicyclo (2,2,1)-5-heptene compound, and U.S. Patent No. 3,752,839 discloses examples using a zero-valent palladium catalyst and zinc chloride catalyst system.
(v) Examples using a zero-valent nickel complex and zinc chloride are disclosed in U.S. patent Nos. 3,850,973 and 3,925,445 which are directed to a process for producing cyanoolefins by hydrocyanation of butadiene and bicyclo (2,2,1)-2,5-heptadiene, and in U.S. patent No. 4,215,068 which is directed to a process for producing a dicyanocyclopentane by hydrocyanation of cyclopentadiene and then hydrocyanation of the resulting product, cyanocyclopentene.

However, it can not be said that prior art has proposed a satisfactory process for producing NDC's.

For example, in (i) above, the yield of NDC's is only about 62 % when a cobalt catalyst and triphenyl phosphine are used in an amount of 15 - 30 % by weight based on BHC and hydrogen cyanide is used in a large amount, that is, 1.4 time that of BHC followed by carrying out the reaction at 130°C for 8 hours.

According to the production process of (ii) above, since an expensive palladium catalyst is used, recovery of the catalyst is troublesome, and the reaction temperature is so high (150 - 190°C) that handling is difficult, and further, as is clear from the low yield, the catalytic activity is low.

In the hydrocyanation of olefins and dienes in (iii) and (v), a zero-valent nickel complex is used in a large amount, i.e. about 0.5 - 2.5 mol % based on the olefin while the conversion of olefins and dienes is 30 - 80 % and the yield of the end product, dinitriles, is as low as 30 - 70 %. In addition, products and yields are not disclosed in the hydrocyanation of bicyclo (2,2,1)-5-heptene compounds in (iv) above.

An object of the present invention is to solve the above-mentioned problems of the prior art and produce NDC's at a high conversion, high selectivity and advantageously from an economical point of view.

### Disclosure of Invention

The present invention is a process for producing a norcamphane dicarbonitrile (NDC) comprising hydrocyanation of bicyclo [2,2,1]-5-heptene-2-carbonitrile (BHC) in the presence of a zero-valent nickel complex catalyst and a Lewis acid, wherein
the zero-valent nickel complex catalyst is represented by the general formula (I),

Ni [(A)(B)(C)(D)] (I)

where A,B,C and D are, similar or dissimilar, and are neutral ligands of formula (II),

P (x)(y)(z) (II)

where P is a phosphorus, x, y and z are, similar or dissimilar, OR where R is selected from the group consisting of alkyl having 18 carbon atoms or less and aryl having 18 carbon atoms or less.

BHC used in the present invention may be easily obtained in a high yield by the Diels-Alder reaction of cyclopentadiene with acrylonitrile by heating, and it is preferable to use a product obtained by purifying the reaction product by distillation or the like.

Examples of the neutral ligands include triaryl phosphites such as triphenyl phosphite; tri-substituted phenyl phosphite such as tri-halo substituted phenyl phosphite, tri-alkoxy substituted phenyl phosphite, tri-alkyl substituted phenyl phosphite; and trialkyl phosphites, and mixtures thereof.

Concrete examples of tri-substituted phenyl phosphites include tri-m- or p-tolyl phosphite, tri-m or p-chlorophenyl phosphite, tri-m or p-methoxyphenyl phosphite, and tri-m or p-nonylphenyl phosphite. Concrete examples of trialkyl phosphites include triethyl phosphite, triisopropyl phosphite, and tributyl phosphite.

One or more of the neutral ligands, A, B, C and D can leave the zero-valent nickel complex catalyst under most reaction conditions.

The neutral ligands are preferably triaryl phosphites, in particular, triphenyl phosphite, tri-m or p-tolyl phosphite, and tri-m or p-nonylphenyl phosphite.

Exemplary suitable zero-valent nickel complex catalysts include tetrakis (triphenyl phosphite) nickel; tetrakis (tri-substituted phenyl phosphite) nickels, for example, tetrakis (tri-halo-substituted phenyl phosphite) nickel, tetrakis (tri-alkoxy-substituted phenyl phosphite) nickel and tetrakis (tri-alkyl-substituted phenyl phosphite) nickel; and tetrakis (trialkyl phosphite) nickel.

As concrete examples of tetrakis (tri-substituted phenyl phosphite) nickels are those where the tri-substituted phenyl phosphite is selected from tri-m- or p-tolyl phosphite, tri-m- or p-chlorophenyl phosphite, tri-m- or p-methoxyphenyl phosphite, tri-m- or p-nonylphenyl phosphite.

Further, exemplary suitable zero-valent nickel complex catalysts include tetrakis (trialkyl phosphite) nickel, for example, tetrakis (triethyl phosphite) nickel, tetrakis (triisopropyl phosphite) nickel, and tetrakis (tributyl phosphite) nickel.

In the present invention, it is preferable to carry out hydrocyanation in the presence of a neutral ligand so as to enhance the activity of the zero-valent nickel complex catalyst and prolong the life of the catalyst.

The amount of the neutral ligand used is usually one mole or more, preferably 2 - 32 moles, more preferably 4 - 16 moles based on one mole of the existing zero-valent nickel complex catalyst. An amount exceeding 32 moles does not adversely affect the reaction itself, but is not always economically preferable taking into consideration the post-treatment of the reaction product fluid and the loss of the neutral ligand upon the purification and recovery procedure.

Taking into account the neutral ligand coordinate in the zero-valent nickel complex, the amount of the total neutral ligand used is usually 5 moles or more, preferably 6 - 36 moles, more preferably 8 - 20 moles per mole of the zero-valent nickel complex catalyst.

Preparation of the zero-valent nickel complex catalyst is disclosed, for example, in U.S. Patent No. 3,328,443, J. Chem. Soc. London, 1378 - 1389 (1960), J. Am. Chem. Soc., 81, 4200 - 4209 (1959) and Inorg. Synth., 13, 108 or 112.

The amount of the zero-valent nickel complex catalyst used in terms of the molar ratio of zero-valent nickel complex catalyst to BHC is usually from 1 : 5000 to 1 : 20, preferably from 1 : 2000 to 1 : 100. When the molar ratio exceeds 1 : 20, there is not obtained an advantage corresponding to the increase in the amount of the catalyst and thereby it is not economical.

In the present invention, a Lewis acid is present as a promoter. As a Lewis acid, there is, for example, a compound comprising a center metal having a vacant orbit.

Examples of Lewis acids are compounds composed of an anion and a metallic cation selected from elements of groups IIa, IIIa, IVa, Va, VIa, VIIa, VIII, Ib, IIb, IIIb, and IVb of the Periodic Table.

Examples of the metallic cation are zinc, cadmium, beryllium, aluminum, gallium, indium, silver, titanium, zirconium, hafnium, germanium, tin, vanadium, niobium, scandium, chromium, molybdenum, tungsten, manganese, rhenium, palladium, thorium, erbium, iron, cobalt, and boron ions.

Examples of anions are halogen anions such as chlorine, bromine, fluorine and iodine, anions of lower fatty acids of C₂-C₇, HPO₃²⁻, H₂PO₂⁻, CF₃CO₂⁻, OSO₂C₇F₁₅⁻, and SO₄²⁻.

Particularly preferable metallic cations are zinc, cadmium, titanium, tin, vanadium, chromium and aluminum ions and examples of anions are chlorine ion, iodine ion, HPO₃²⁻, and H₂PO₂⁻.

In addition, examples of a Lewis acid are organoboron compounds, for example, trialkyl boron such as triethyl boron, triphenyl boron and metal alkoxides such as aluminum isopropoxide and titanium isopropoxide.

Examples of preferable Lewis acids are zinc chloride, cadmium chloride, cadmium iodide, chromium chloride, boron trichloride, and triphenyl boron, and zinc chloride is particularly preferable.

A Lewis acid as a promoter can prolong the life of the catalyst and the amount of Lewis acid used is usually 0.05 - 50 moles, preferably 0.5 - 5 moles per mole of the zero-valent nickel complex catalyst.

In the hydrocyanation of the present invention, the neutral ligand can behave as a solvent, but additionally other solvent or solvents may be used.

The solvents used include, for example, aryl compounds containing at least one hydroxyl group and having 6 - 20 carbon atoms, preferably, 6 - 10 carbon atoms, and if desired, may be the above-mentioned aryl compounds having at least one substituent selected from the group consisting of fluoro, chloro, bromo, iodo, nitro, cyano, and hydrocarbon groups having 1 - 9 carbon atoms. The aryl compounds are, for example, phenol, p-cresol, resorcinol, β-naphthol, p-chlorophenol, p-nitrophenol, p-butylphenol and analogs thereof.

As other solvents, there may be mentioned, for example, aromatic hydrocarbons such as benzene, toluene, xylene, ethylbenzene and the like; nitriles such as acetonitrile, benzonitrile; ethers such as dioxane, o-dimethoxybenzene, tetrahydrofuran, dimethoxyethane, diethoxyethane and the like; chloroaromatic hydrocarbons such as o-dichlorobenzene, p-dichlorobenzene and analogs thereof.

According to the present invention, the hydrocyanation of BHC can be effected, for example, such that prescribed amounts of the above-mentioned zero-valent nickel complex catalyst, Lewis acid, BHC, and neutral ligand (or solvent) are fed to a reactor and then hydrogen cyanide is introduced into the reaction fluid under stirring at a prescribed temperature. Hydrogen cyanide may be introduced thereinto in the form of either gaseous hydrogen cyanide or liquid hydrogen cyanide.

Hydrogen cyanide may be used alone, but, from the standpoint of handling, it is desirable to dilute hydrogen cyanide to an appropriate concentration with a gas inert to the reaction such as nitrogen, helium and argon.

Liquid hydrogen cyanide may be used alone or may be diluted with a solvent such as benzene, xylene and the like to an appropriate concentration for use.

Hydrogen cyanide is used usually in an amount of 0.2 - 1.5 mole, preferably 0.5 - 1.2 mole per mole of BHC.

The reaction temperature of hydrocyanation of BHC is usually in the range of from -20 to 200°C, preferably from 20 to 130°C, more preferably from 50 to 100°C.

The reaction pressure is, in usual, preferably atmospheric pressure, and the reaction may be effected at a pressurized system, but there is not any remarkable advantage due to the high pressure.

The hydrocyanation of BHC is usually effected batchwise, but a continuous reaction system may be employed such that BHC, hydrogen cyanide, zero-valent nickel complex catalyst, Lewis acid, neutral ligand and the like are continuously fed to a reactor.

NDC's produced by the present invention can be obtained as a mixture containing 2,5-NDC and 2,6-NDC as the components.

After completion of the reaction, the resulting reaction product fluid containing NDC's produced by hydrocyanation of BHC at a high concentration is subjected to a post-treatment, for example, for recoverying effective components as catalyst and the like, that is, extraction with an organic solvent or extracting Lewis acids and inorganic materials with water, and then NDC's are obtained by distillation.

Distillation of NDC's is preferably carried out at a pressure of 0.3 - 0.5 mm Hg to collect a distillate fraction at 120 - 130°C as NDC's.

According to the present invention, NDC's can be produced at high conversion and at high selectivity economically by hydrocyanation of BHC in the presence of a zero-valent nickel complex catalyst and a promoter system composed of a Lewis acid, and therefore, the process for producing NDC's is very advantageous.

### Best Mode for Carrying Out the Invention

For the purpose of describing the present invention more in detail, the following examples are given. Comparative examples are also given to help the understanding of the present invention.

Analysis of the reaction fluid was conducted by gas chromatography.

### EXAMPLE 1

A 50 ml. glass round-bottom flask fitted with a stirrer, a thermometer, a gas inlet tube, a cooler and the like was charged with BHC 27.55 g (229 m mol), tetrakis (triphenyl phosphite) nickel 0.79 g (0.608 m mol), zinc chloride 0.44 g (3.2 m mol) and triphenyl phosphite 3.10 g (10.0 m mol), purged with a nitrogen gas sufficiently, and the contents in the reactor was kept at 95°C with stirring.

Then, nitrogen gas was introduced into a receiving vessel containing liquid hydrogen cyanide cooled with ice water and bubbled through the liquid hydrogen cyanide to generate a hydrogen cyanide gas and said gas was introduced into the reaction fluid in the reactor.

The flow rate of nitrogen gas was 55 - 60 ml/min. 7.0 g (259 m mol) of hydrogen cyanide gas in total was fed to the reactor over 5 hours and then the reaction was completed.

The reaction fluid was cooled and analyzed, and it was found that the conversion of BHC was 97.5 % and selectivity of NDC's 94.4 %.

### EXAMPLES 2 - 6

The procedure of Example 1 was repeated using the same reactor as in Example 1 except that molar ratio of BHC, tetrakis (triphenyl phosphite) nickel, zinc chloride, triphenyl phosphite and hydrogen cyanide, temperature and feeding time of hydrogen cyanide gas were changed.

The results are shown in Table 1.

### EXAMPLE 7

The reaction of Example 1 was repeated except that liquid hydrogen cyanide cooled with ice was directly fed to the reactor using a microtube pump in place of the hydrogen cyanide gas.

The flow rate of liquid hydrogen cyanide was 2 - 2.5 ml/hr, and 7.0 g (259 m mol) of liquid hydrogen cyanide in total was fed to the reactor over 5 hours and the reaction was completed.

The reaction fluid was cooled and analyzed. It was found that the conversion of BHC was 99.9 % and selectivity of NDC's 99.8 % % (see Table 2).

### EXAMPLES 8 - 9

The procedure of Example 7 was repeated except that the molar ratio of liquid hydrogen cyanide, temperature, and feeding time of hydrogen cyanide were changed.

The results are shown in Table 2.

### EXAMPLES 10 - 13

The procedure of Example 6 was repeated with the same feed as in Example 6 except that the zero-valent nickel complex catalyst and the neutral ligand were replaced with the catalyst systems shown in Table 3. The results are shown in Table 3.

### EXAMPLES 14 - 18

The hydrocyanation of Example 6 was repeated with the same feed as in Example 6 except that Lewis acids in Table 4 were used in place of zinc chloride. The results are shown in Table 4.

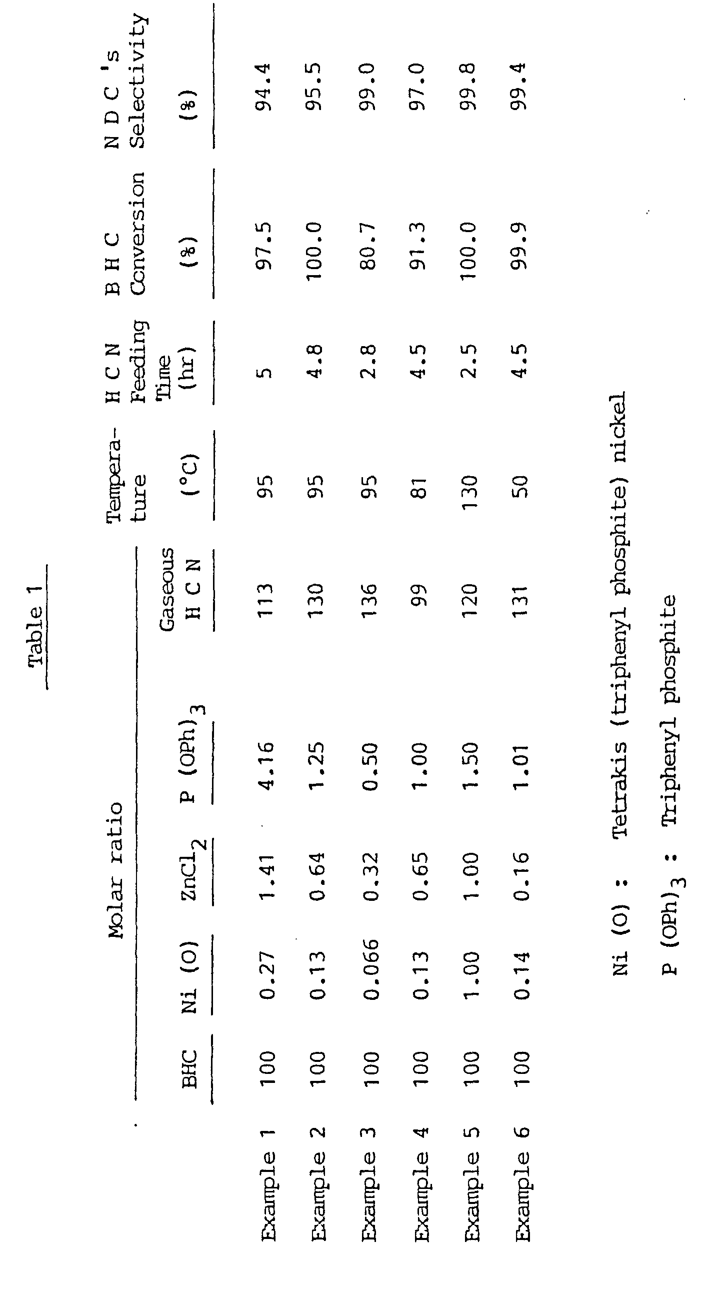

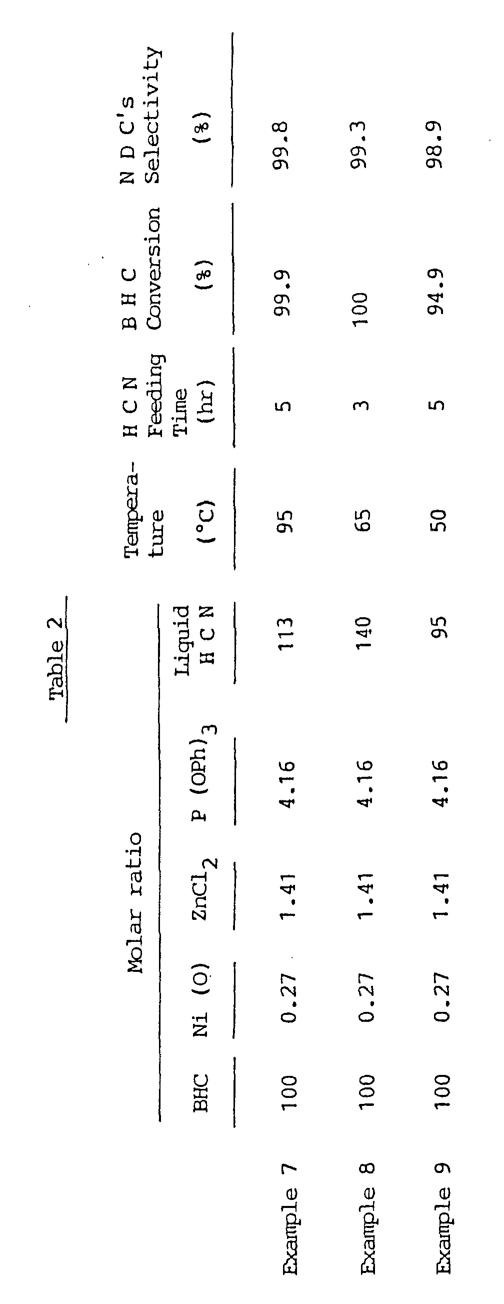

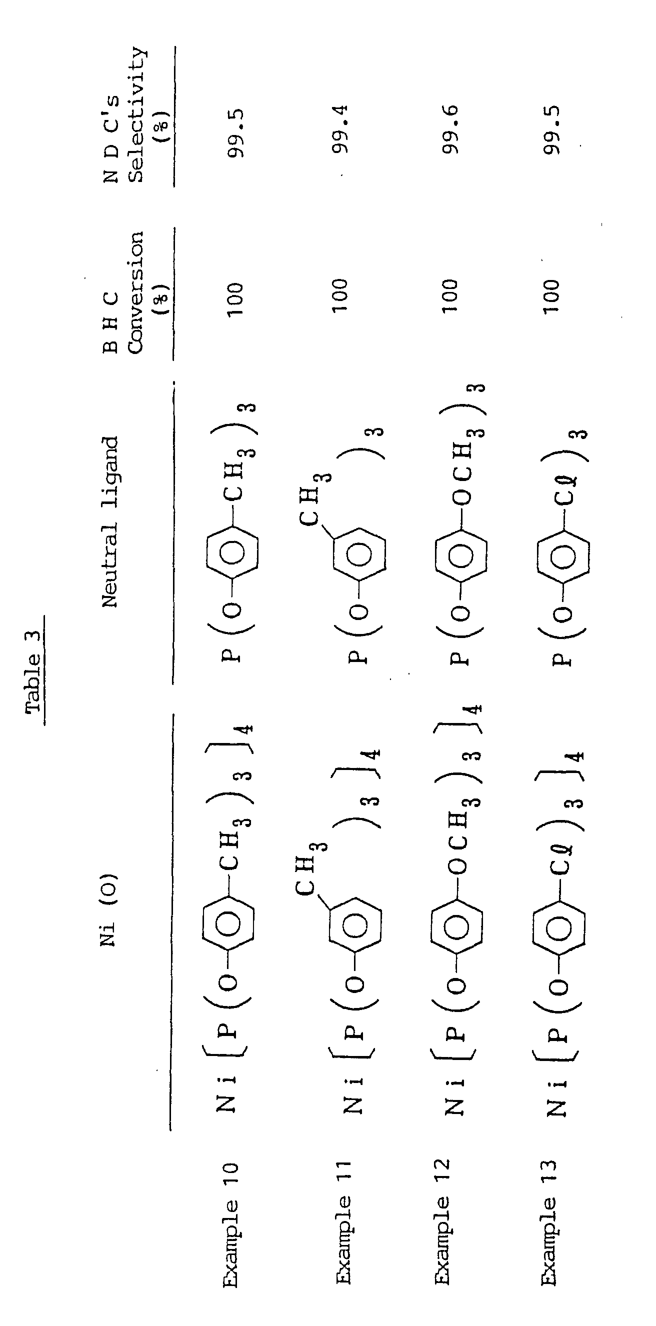

**Table 4**

| | Lewis acid | B H C Conversion (%) | N D C's Selectivity (%) |
|---|---|---|---|
| Example 14 | SnCl₂ | 97.2 | 99.3 |
| Example 15 | CdCl₃ | 98.5 | 99.7 |
| Example 16 | CrCl₃ | 95.6 | 99.5 |
| Example 17 | Al (O-iPr)₃ | 85.9 | 99.0 |
| Example 18 | BPh₃ | 99.9 | 99.8 |
| Al (O-iPr)₃ : Aluminum isopropoxide | | | |
| BPh₃ : Triphenyl boron | | | |

### COMPARATIVE EXAMPLE 1

The reaction of Example 1 was repeated with the same feed as in Example 1 except that tetrakis (triphenyl phosphite) nickel was replaced with 0.82 g (0.608 m mol) of tetrakis (triphenyl phosphite) palladium. As a result, the conversion of BHC was 31.6 % and the selectivity of NDC's 93.1 %.

### COMPARATIVE EXAMPLE 2

The reaction of Example 6 was repeated with the same feed as in Example 6 except that zinc chloride was not used.

As a result, the conversion of BHC was 4.1 % and the selectivity of NDC's 21.5 %.

### Industrial Applicability

NDC's produced by the present invention can be hydrogenated to prepare the corresponding diamines, and the diamines may be reacted with aliphatic dicarboxylic acids to produce polyamide resins. Therefore, the NDC's are useful as intermediates for organic syntheses.

## Claims

1. A process for producing a norcamphane dicarbonitrile which comprises hydrocyanation of bicyclo [2,2,1]-5-heptene-2-carbonitrile in the presence of a zero-valent nickel complex catalyst and a Lewis acid, wherein the zero-valent nickel complex catalyst is represented by the general formula (I),
Ni [ (A) (B) (C) (D) ] (I)
where A, B, C and D are, similar or dissimilar, neutral ligands of formula (II),
P (x) (y) (z) (II)
where P is a phosphorus atom, x, y and z are similar or dissimilar groups represented by OR where R is selected from the group consisting of alkyl having 18 carbon atoms or less and aryl having 18 carbon atoms or less.

2. A process according to claim 1 wherein hydrocyanation is effected in the presence of a neutral ligand.

3. A process according to claim 2 wherein the neutral ligand is a compound of the general formula (II),
P (x) (y) (z) (II)
where P is a phosphorus atom and x, y and z are similar or dissimilar groups represented by OR where R is selected from the group consisting of alkyl having 18 carbon atoms or less and aryl having 18 carbon atoms or less.

4. A process according to claim 1 wherein the zero-valent nickel complex catalyst is tetrakis (triaryl phosphite) nickel, particularly tetrakis (triphenyl phosphite) nickel or tetrakis (trialkyl phosphite) nickel particularly tetrakis (tri-substituted phenyl phosphite) nickel.

5. A process according to claim 4 wherein the tetrakis (tri-substituted phenyl phosphite) nickel is a member selected from the group consisting of tetrakis (tri-halo-substituted phenyl phosphite) nickel, tetrakis (tri-alkoxy-substituted phenyl phosphite) nickel and tetrakis (tri-alkyl-substituted phenyl phosphite) nickel.

6. A process according to claim 5, wherein the tetrakis (tri-substituted phenyl phosphite) nickel is a member selected from the group consisting of tetrakis (tri-m or p-tolyl phosphite) nickel, tetrakis (tri-m or p-chlorophenyl phosphite) nickel, tetrakis (tri-m or p-methoxyphenyl phosphite) nickel and tetrakis (tri-m or p-nonylphenyl phosphite) nickel.

7. A process according to claim 4 wherein the tetrakis (trialkyl phosphite) nickel is a member selected from the group consisting of tetrakis (triethyl phosphite) nickel, tetrakis (triisopropyl phosphite) nickel and tetrakis (tributyl phosphite) nickel.

8. A process according to claim 1 or claim 2 wherein the Lewis acid is a compound composed of an anion and a metal cation of an element selected from the group consisting of groups IIa, IIIa, IVa, Va, VIa, VIIa, VIII, Ib, IIb, IIIb, and IVb of the Periodic Table.

9. A process according to claim 8 wherein the Lewis acid contains a cation of a metal selected from the group consisting of zinc, cadmium, beryllium, aluminium, gallium, indium, silver, titanium, zirconium, hafnium, germanium, tin, vanadium, niobium, scandium, chromium, molybdenum, tungsten, manganese, rhenium, palladium, thorium, erbium, iron, cobalt, and boron.

10. A process according to claim 8 or 9 wherein the anion of the Lewis acid is an anion selected from the group consisting of halogen anions, that is, chlorine, bromine, fluorine and iodine, anions of lower fatty acid of C₂-C₇, HPO₃²⁻, H₂PO₂⁻, CF₃CO₂⁻,OSO₂C₇F₁₅⁻, and SO₄²⁻.

11. A process according to claim 1 or claim 2 wherein the Lewis acid is a member selected from the group consisting of zinc chloride, cadmium chloride, cadmium iodide, chromium chloride, boron trichloride and triphenyl boron.

12. A process according to claim 1 or claim 2 wherein the Lewis acid is organoboron compound or metal alkoxide.

13. A process according to claim 12 wherein the Lewis acid is a member selected from the group consisting or trialkyl boron, triphenyl boron, aluminum alkoxide and titanium alkoxide.

14. A process according to claim 1 or claim 2 wherein the molar ratio of the zero-valent nickel complex catalyst to bicyclo [2,2,1]-5-heptene-2-carbonitrile ranges from 1 : 5000 to 1 : 20., particularly from 1 : 2000 to 1 : 100.

15. A process according to claim 1 or claim 2 wherein the molar ratio of the Lewis acid to the zero-valent nickel complex catalyst ranges from 0.05 : 1 to 50 : 1, particularly from 0.5 : 1 to 5 : 1.

16. A process according to claim 2 wherein the total neutral ligand per mole of the zero-valent nickel complex catalyst is 5 moles or more, preferably 6 - 36 moles, particularly 8 - 20 moles.

17. A process according to claim 1 or claim 2 wherein the reaction temperature of hydrocyanation is in the range of from -20 to 200°C, preferably 20 - 130°C, particularly 50 - 100°C.

18. A process according to claim 1 or claim 2 wherein hydrogen cyanide is introduced in the form of gas or liquid.

19. A process according to claim 1 or claim 2 wherein the molar ratio of hydrogen cyanide to bicyclo [2.2.1]-5-heptene-2-carbonitrile is in the range of from 0.2 : 1 to 1.5 : 1, particularly from 0.5 : 1 to 1.2 : 1.

## Patentansprüche

1. Verfahren zur Herstellung eines Norbornandicarbonitrils, welches Verfahren eine Hydrocyanierung von Bicyclo[2.2.1]-5-hepten-2-carbonitrils in Gegenwart eines 0-wertigen Nickelkomplex-Katalysators und einer LEWIS-Säure umfaßt, wobei sich der 0-wertige Nickelkomplex-Katalysator darstellen läßt durch die allgemeine Formel (I),
Ni [ (A) (B) (C) (D) ] (I)
worin A, B, C und D gleichartige oder verschiedene neutrale Liganden der Formel (II) sind,
P (x) (y) (z) (II)
worin P ein Phosphoratom ist und x, y und z gleichartige oder verschiedene Gruppen sind, die sich durch OR wiedergeben lassen, wobei R ausgewählt ist aus der Gruppe, bestehend aus Alkyl mit 18 oder weniger Kohlenstoffatomen und Aryl mit 18 oder weniger Kohlenstoffatomen.

2. Verfahren nach Anspruch 1, in welchem die Hydrocyanierung in Gegenwart eines neutralen Liganden erfolgt.

3. Verfahren nach Anspruch 2, in welchem der neutrale Ligand eine Verbindung der allgemeinen Formel (II) ist,
P (x) (y) (z) (II)
worin P ein Phosphoratom ist und x, y und z gleichartige oder verschiedene Gruppen sind, die sich durch OR wiedergeben lassen, wobei R ausgewählt ist aus der Gruppe, bestehend aus Alkyl mit 18 oder weniger Kohlenstoffatomen und Aryl mit 18 oder weniger Kohlenstoffatomen.

4. Verfahren nach Anspruch 1, in welchem der 0-wertige Nickelkomplex-Katalysator ein Tetrakis(triarylphosphit)Nickel, insbesondere Tetrakis(triphenylphosphit)Nickel oder Tetrakis(trialkylphosphit)Nickel, insbesondere Tetrakis(tri-substituierter Phenylphosphit) Nickel ist.

5. Verfahren nach Anspruch 4, in welchem der Tetrakis(tri-substituierte Phenylphosphit) Nickel ausgewählt ist aus der Gruppe Tetrakis(trihalogen-substituierter Phenylphosphit)Nickel, Tetrakis(trialkoxy-substituierter Phenylphosphit)Nickel oder Tetrakis(trialkyl-substituierter Phenylphosphit)Nickel.

6. Verfahren nach Anspruch 5, in welchem der Tetrakis(tri-substituierte Phenylphosphit) Nickel ausgewählt ist aus der Gruppe Tetrakis(tri-m- oder p-tolylphosphit)Nickel, Tetrakis(tri- m- oder p-chlorphenylphosphit)Nickel, Tetrakis(tri-m- oder p-methoxyphenylphosphit)Nickel und Tetrakis(tri-m- oder p-nonylphenylphosphit)Nickel.

7. Verfahren nach Anspruch 4, in welchem der Tetrakis(trialkylphosphit)Nickel ausgewählt ist aus der Gruppe Tetrakis(triethylphosphi)Nickel, Tetrakis(triisopropylphosphit)Nickel und oder Tetrakis(tributylphosphit)Nickel.

8. Verfahren nach Anspruch 1 oder 2, in welchem die LEWIS-Säure eine Verbindung ist, die zusammengesetzt ist aus einem Anion und einem Metallkation eines aus den Gruppen IIa, IIIa, IVa, Va, VIa, VIIa, VIII, Ib, IIb, IIIb, und IVb des Periodensystems ausgewählten Elements.

9. Verfahren nach Anspruch 8, in welchem die LEWIS-Säure ein Kation aus einem Metall enthält, das ausgewählt ist aus der Gruppe Zink, Cadmium, Beryllium, Aluminium, Gallium, Indium, Silber, Titan, Zirkon, Hafnium, Germanium, Zinn, Vanadium, Niob, Scandium, Chrom, Molybdän, Wolfram, Mangan, Rhenium, Palladium, Thorium, Erbium, Eisen, Kobalt und Bor.

10. Verfahren nach Anspruch 8 oder 9, in welchem das Anion der LEWIS-Säure ein aus der Gruppe Halogenanionen, d.h. Chlor-, Brom-, Fluor- und Iodanionen niedriger Fettsäuren von C₂-C₇, HPO₃²⁻,H₂PO²⁻, CF₃CO₂⁻,OSO₂C₇F₁₅⁻ und SO₄²⁻ ausgesuchtes Anion ist.

11. Verfahren nach Anspruch 1 oder 2, in welchem die LEWIS-Säure ausgesucht ist aus der Gruppe Zinkchlorid, Cadmiumchlorid, Cadmiumiodid, Chromchlorid, Bortrichlorid und Triphenylbor.

12. Verfahren nach Anspruch 1 oder 2, in welchem die LEWIS-Säure eine Organobor-Verbindung oder ein Metallalkoxid ist.

13. Verfahren nach Anspruch 12, in welchem die LEWIS-Säure ausgewählt ist aus der Gruppe Trialkylbor, Triphenylbor, Aluminiumalkoxid und Trititanalkoxid.

14. Verfahren nach Anspruch 1 oder 2, in welchem das molare Verhältnis des 0-wertigen Nickelkomplex-Katalysators zum Bicyclo[2,2,1]-5-hepten-2-carbonitril im Bereich von 1 : 5000 bis 1 : 20, insbesondere 1 : 2000 bis 1 : 100, liegt.

15. Verfahren nach Anspruch 1 oder 2, in welchem das molare Verhältnis der LEWIS-Säure zum 0-werigen Nickelkomplex-Katalysator im Bereich von 0,05 : 1 bis 50 : 1, insbesondere im Bereich von 0,5 : 1 bis 5 : 1, liegt.

16. Verfahren nach Anspruch 2, in welchem der Gesamtgehalt an neutralem Liganden pro Mol des 0-wertigen Nickelkomplex-Katalysators 5 Mol oder mehr, vorzugsweise 6 - 36 Mol, insbesondere 8 - 20 Mol, beträgt.

17. Verfahren nach Anspruch 1 oder 2, in welchem die Reaktionstemperatur bei der Hydrocyanierung im Bereich von -20 bis 200°C, vorzugsweise von 20 bis 130°C, insbesondere von 50 bis 100°C liegt.

18. Verfahren nach Anspruch 1 oder 2, in welchem der Cyanwasserstoff in Form eines Gases oder einer Flüssigkeit zugesetzt wird.

19. Verfahren nach Anspruch 1 oder 2, in welchem das molare Verhältnis des Cyanwasserstoffs zum Bicyclo[2,2,1]-5-hepten-2-carbodinitril im Bereich von 0,2 : 1 bis 1,5 : 1, insbesondere von 0,5 : 1 bis 1,2 : 1, liegt.

## Revendications

1. Procédé de production d'un norcamphane dicarbonitrile, qui comprend l'hydrocyanation de bicyclo[2,2,1]-5-heptène-2-carbonitrile en présence d'un catalyseur de complexe de nickel de valence zéro et d'un acide de Lewis, dans lequel le catalyseur de complexe de nickel de valence zéro est représenté par la formule générale (I):
Ni[(A)(B)(C)(D)] (I)
dans laquelle A, B, C et D sont des ligands neutres, semblables ou dissemblables de la formule (II) :
P(x)(y)(z) (II)
dans laquelle P est un atome de phosphore, x, y et z sont des groupes semblables ou dissemblables représentés par OR dans lequel R est choisi parmi les groupes alkyle comportant 18 atomes de carbone ou moins et les groupes aryle comportant 18 atomes de carbone ou moins.

2. Procédé suivant la revendication 1, dans lequel l'hydrocyanation est effectuée en présence d'un ligand neutre.

3. Procédé suivant la revendication 2, dans lequel le ligand neutre est un composé de la formule générale (II) :
P(x)(y)(z) (II)
dans laquelle P est un atome de phosphore, x, y et z sont des groupes semblables ou dissemblables représentés par OR dans lequel R est choisi parmi les groupes alkyle comportant 18 atomes de carbone ou moins et les groupes aryle comportant 18 atomes de carbone ou moins.

4. Procédé suivant la revendication 1, dans lequel le catalyseur de complexe de nickel de valence zéro est un tétrakis triaryl phosphite) nickel, en particulier le tétrakis (triphényl phosphite) nickel, ou un tétrakis (trialkyl phosphite) nickel, en particulier un tétrakis (phényl phosphite trisubstitué) nickel.

5. Procédé suivant la revendication 4, dans lequel le tétrakis (phényl phosphite trisubstitué) nickel est un membre du groupe comprenant les tétrakis (phényl phosphite trihalo-substitué) nickels, les tétrakis (phényl phosphite trialcoxy-substitué) nickels et les tétrakis (phényl phosphite trialkyl-substitué) nickels.

6. Procédé suivant la revendication 5, dans lequel le tétrakis (phényl phosphite trisubstitué) nickel est un membre du groupe comprenant les tétrakis (tri-m- et p-tolyl phosphite) nickels, les tétrakis (tri-m- et p-chlorophényl phosphite) nickels, les tétrakis (tri-m- et p-méthoxyphényl phosphite) nickels et les tétrakis (tri-m- et p-nonylphényl phosphite) nickels.

7. Procédé suivant la revendication 4, dans lequel le tétrakis (trialkyl phosphite) nickel est un membre du groupe comprenant le tétrakis (triéthyl phosphite) nickel, le tétrakis (triisopropyl phosphite) nickel et le tétrakis (tributyl phosphite) nickel.

8. Procédé suivant l'une ou l'autre des revendications 1 et 2, dans lequel l'acide de Lewis est un composé formé d'un anion et d'un cation métallique d'un élément choisi dans le groupe comprenant les Groupes IIa, Illa, IVa, Va, VIa, Vlla, VIII, Ib, IIb, IIIb et IVb du Tableau Périodique.

9. Procédé suivant la revendication 8, dans lequel l'acide de Lewis contient un cation d'un métal choisi dans le groupe comprenant le zinc, le cadmium, le béryllium, l'aluminium, le gallium, l'indium, l'argent, le titane, le zirconium, l'hafnium, le germanium, l'étain, le vanadium, le niobium, le scandium, le chrome, le molybdène, le tungstène, le manganèse, le rhénium, le palladium, le thorium, l'erbium, le fer, le cobalt et le bore.

10. Procédé suivant l'une ou l'autre des revendications 8 et 9, dans lequel l'anion de l'acide de Lewis est un anion choisi dans le groupe comprenant les anions d'halogène, c'est-à-dire le chlore, le brome, le fluor et l'iode, les anions d'acide gras inférieur en C₂-C₇, HPO₃²⁻, H₂PO₂⁻, CF₃CO₂⁻, OSO₂C₇F₁₅⁻ et SO₄²⁻.

11. Procédé suivant l'une ou l'autre des revendications 1 et 2, dans lequel l'acide de Lewis est un membre du groupe comprenant le chlorure de zinc, le chlorure de cadmium, l'iodure de cadmium, le chlorure de chrome, le trichlorure de bore et le triphényl bore.

12. Procédé suivant l'une ou l'autre des revendications 1 et 2, dans lequel l'acide de Lewis est un composé organoboré ou un alcoolate métallique.

13. Procédé suivant la revendication 12, dans lequel l'acide de Lewis est un membre du groupe comprenant les trialkyl bores, le triphényl bore, l'alcoolate d'aluminium et l'alcoolate de titane.

14. Procédé suivant l'une ou l'autre des revendications 1 et 2, dans lequel le rapport molaire du catalyseur de complexe de nickel de valence zéro au bicyclo[2,2,1]-5-heptène-2-carbonitrile est de 1/5000 à 1/20, en particulier de 1/2000 à 1/100.

15. Procédé suivant l'une ou l'autre des revendications 1 et 2, dans lequel le rapport molaire de l'acide de Lewis au catalyseur de complexe de nickel de valence zéro est de 0,05/1 à 50/1, en particulier de 0,5/1 à 5/1.

16. Procédé suivant la revendication 2, dans lequel le ligand neutre total par mole de catalyseur de complexe de nickel de valence zéro est de 5 moles ou plus, avantageusement de 6-36 moles, en particulier de 8-20 moles.

17. Procédé suivant l'une ou l'autre des revendications 1 et 2, dans lequel la température de réaction de l'hydrocyanation se situe dans l'intervalle de -20 à 200°C, avantageusement de 20-130°C, en particulier de 50-100°C.

18. Procédé suivant l'une ou l'autre des revendications 1 et 2, dans lequel le cyanure d'hydrogène est introduit sous la forme de gaz ou de liquide.

19. Procédé suivant l'une ou l'autre des revendications 1 et 2, dans lequel le rapport molaire du cyanure d'hydrogène au bicyclo[2,2,1]-5-heptène-2-carbonitrile se situe dans la gamme de 0,2/1 à 1,5/1, en particulier de 0,5/1 à 1,2/1.
